# EUROPÄISCHE PATENTSCHRIFT

(11) **EP 1 192 894 B1**
(45) Veröffentlichungstag und Bekanntmachung des Hinweises auf die Patenterteilung: **13.10.2004**
(21) Anmeldenummer: 00810866.4
(22) Anmeldetag: 21.09.2000
(51) Int. Cl.: A61B 3/024, A61B 3/00

(54) **Perimeter für die Gesichtfelduntersuchung**
Visual perimetry testing device
Appareil de périmétrie du champ visuel

(43) Veröffentlichungstag der Anmeldung: 03.04.2002
(73) Patentinhaber: Haag-Streit AG, 3098 Köniz (CH)
(72) Erfinder: Hansueli, Walther, 5400 Baden (CH)
(74) Vertreter: Groner, Manfred

(56) Entgegenhaltungen:
- EP-A- 0 850 591
- WO-A-00/13571
- GB-A- 326 053
- US-A- 2 855 821
- US-A- 5 387 952
- US-A- 5 903 336
- US-A- 6 045 227

## Beschreibung

Die Erfindung betrifft ein Perimeter für die Gesichtsfelduntersuchung nach dem Oberbegriff des Anspruchs 1.

Automatische Perimeter für die Gesichtsfelduntersuchung sind bekannt und werden sowohl in der Forschung als auch für Routineuntersuchungen in der Praxis eingesetzt. Ein solches Perimeter ist neben anderen unter dem Warenzeichen OCTOPUS bekannt geworden. Mit diesem Perimeter wird die Lichtunterschiedsempfindlichkeit der Netzhaut an verschiedenen Orten des Gesichtsfeldes computergesteuert ermittelt. Für solche Untersuchungen ist es wesentlich, dass der Patient seinen Kopf in einer vorbestimmten Position hält, welche für das linke und das rechte Auge bezüglich einer Beobachtungsachse symmetrisch ist. Dabei sitzt der Patient vor dem Perimeter und mittels einer Kopfstütze, im Wesentlichen bestehend aus einer Kinn- und einer Stirnstütze, wird der Kopf bzw. das untersuchte Auge vor dem Okular positioniert. Gegenüber dem Patienten sitzt die Bedienungsperson und bedient das Perimeter mittels eines Bedienungspanels, das am Perimeter angebracht ist.

Durch die US-5,125,731 ist ein Perimeter der genannten Art bekannt geworden, bei welchem die Stirn- und die Kinnstütze zur Positionierung des Kopfes bzw. des untersuchten Auges eines Patienten während der Untersuchung verstellbar sind. Bei einer solchen Feinpositionierung wird jedoch der Patient in seiner Konzentration gestört, was sich unweigerlich negativ auf die Untersuchungsdauer und auf die Qualität der Untersuchungsergebnisse auswirkt. Perimeter haben zudem ganz allgemein die Eigenschaft, dass diese voluminös sind und eine geeignete Positionierung in einem Untersuchungsraum häufig schwierig ist, wobei zu berücksichtigen ist, dass sowohl für den Patienten als auch für die Bedienungsperson eine geeignete Sitzposition erforderlich ist.

Durch die GB-326,053 A ist ein Gerät zur Augenuntersuchung bekannt geworden, das eine Optikeinheit aufweist, die an einem Gerätefuss verstellbar angeordnet ist. Am Gerätefuss sind zudem eine höhenverstellbare Kinnstütze und eine um eine horizontale Achse schwenkbare Stirnstütze befestigt. Das Gerät soll in ein Perimeter umbaubar sein.

Der Erfindung liegt die Aufgabe zugrunde, ein Perimeter für die Gesichtsfelduntersuchung zu schaffen, das die genannten Nachteile vermeidet, und das durch bequeme Positionierung des Patienten und einfache Bedienung präzise Untersuchungsergebnisse liefert.

Die Erfindung ist bei einem genannten Perimeter gemäss Anspruch 1 gelöst.

Beim erfindungsgemässen Perimeter ist die Optikeinheit beweglich auf dem Gerätefuss gelagert und kann bezüglich des Bedienungspanels verstellt werden. Damit ist es möglich, die Position zwischen dem Patienten und der Bedienungsperson frei zu wählen. Die Bedienungsperson und der Patient sitzen einander nicht mehr gezwungenermassen gegenüber oder in einem anderen definierten Winkel, die Position kann frei gewählt werden, was vor allem bei engen Raumverhältnissen vorteilhaft ist. Ein weiterer Vorteil des erfindungsgemässen Perimeters besteht darin, dass zur Feinpositionierung des untersuchten Auges vor dem Okular die Optikeinheit in allen Richtungen nachführbar ist. Da hierbei die Kopfstütze nicht verstellt werden muss, wird der Patient bei einer solchen Feinpositionierung in seiner Konzentration nicht gestört.

Weitere vorteilhafte Merkmale ergeben sich aus den abhängigen Patentansprüchen, der nachfolgenden Beschreibung sowie der Zeichnungen.
Ein Ausführungsbeispiel der Erfindung wird nachfolgend anhand der Zeichnungen näher erläutert. Es zeigen:
- Fig. 1: schematisch eine räumliche Ansicht eines erfindungsgemässen Perimeters,
- Fig. 2a: schematisch ein Schnitt durch das Perimeter,
- Fig. 2b: ein weiterer Schnitt durch das Perimeter, und
- Fig. 3a bis 3c: schematisch jeweils ein erfindungsgemässes Perimeter mit unterschiedlichen Ausrichtungen der Optikeinheit.

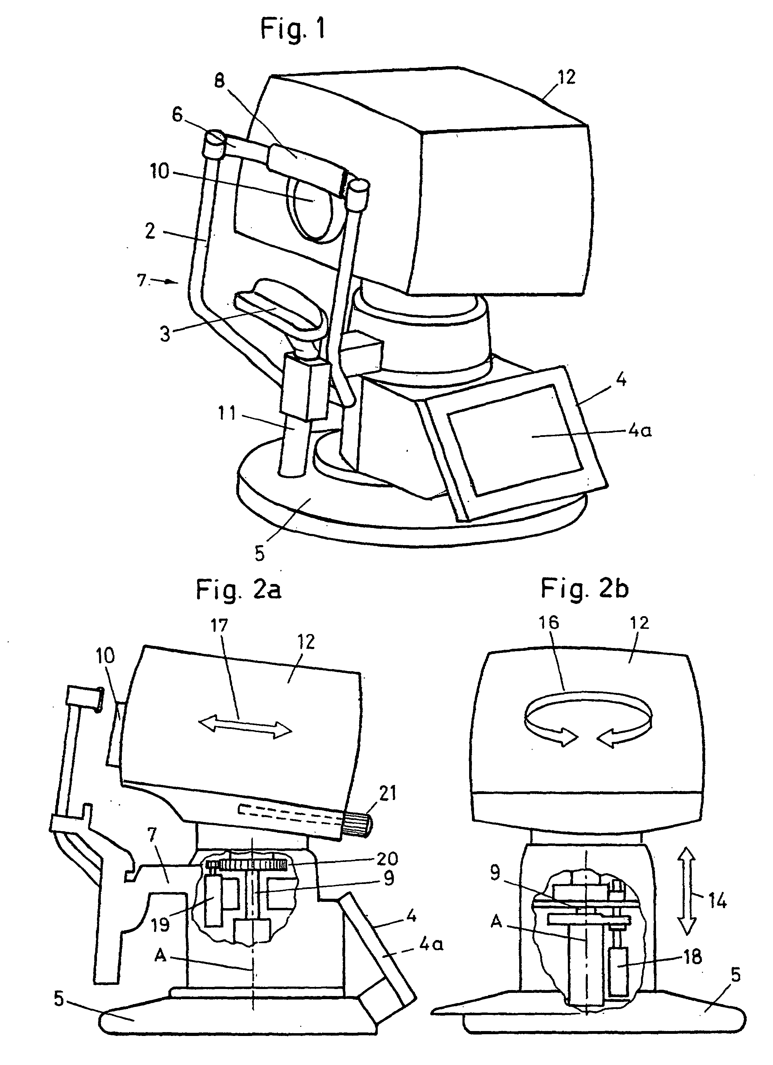

Das Perimeter weist gemäss Fig. 1 einen Gerätefuss 5 auf, der beispielsweise auf einem Tisch positioniert ist. Am Gerätefuss 5 ist eine Kopfstütze 7 drehbar gelagert und ein Bedienungspanel 4 befestigt. Am Bedienungspanel 4 kann die Bedienungsperson das Perimeter bedienen und die Untersuchung überwachen. Hierzu weist das Bedienungspanel 4 eine Anzeige 4a sowie hier nicht gezeigte Tasten und andere Bedienungsmittel auf.

Die Kopfstütze 7 besteht aus einer Kinnstütze 3 und zwei Säulen 2, an denen die Stirnstütze 6 befestigt ist. In der Stirnstütze 6 sind Sensoren 8 integriert, die die Position des Kopfes automatisch feststellen. Die Kinnstütze 3 ist mittels einer Spindel 11 in der Höhe verstellbar, die Kopfstütze 7 ihrerseits kann um den Gerätefuss 5 gedreht werden. Der Patient positioniert seinen Kopf auf dieser Kopfstütze 7 so, dass das linke oder das rechte Auge bezüglich des Okulars 10 und der Beobachtungsachse genau positioniert ist.

Im Gerätefuss 5 ist eine Säule 9 gelagert, die vertikal verstellbar ist und auf welcher eine Optikeinheit 12 angebaut ist, die um die Säule 9 geschwenkt und in Richtung der Beobachtungsachse bewegt werden kann. In der Optikeinheit 12 ist die an sich bekannte und hier nicht gezeigte Untersuchungsoptik mit dem Okular 10 untergebracht.

Die Fig. 2a und 2b zeigen mit dem Doppelpfeil 14 die vertikale Verstellbarkeit der Hubsäule 9 und damit der Optikeinheit 12. Der Doppelpfeil 16 zeigt die Schwenkbarkeit der Optikeinheit 12 auf der Hubsäule 9 und mit dem Doppelpfeil 17 die Verschiebung der Optikeinheit 12 in Richtung der Beobachtungsachse. Zur Höhenverstellung der Hubsäule 9 ist im Gerätefuss 5 ein Antrieb 18 angeordnet. Die Schwenkbewegung der Optikeinheit 12 in Richtung des Doppelpfeils 16 erfolgt mit einem Antrieb 19 über ein Getriebe 20. Die Verschiebung der Optikeinheit 12 in Richtung der Beobachtungsachse 17 erfolgt beispielsweise über eine mechanische Spindel 21. Alle Antriebe können elektrische oder sonstige Antriebe sein. Die Antriebe 18 und 19 sind vom Bedienungspanel 4 aus steuerbar, die Bewegung in Richtung der Beobachtungsachse 17 erfolgt im Ausführungsbeispiel manuell. Die Feinpositionierung wird dadurch kontrolliert, dass das untersuchte Auge des Patienten mit einer Kamera erfasst und das Bild auf der Anzeige 4a dargestellt wird.

Die Beschreibung im obigen Abschnitt erläutert die Bewegungen, die für die Feinpositionierung der Optikeinheit bezüglich des untersuchten Auges des Patienten erforderlich sind. Um die Position zwischen Patient und Bedienungsperson zu verändern, wird die Optikeinheit 12 in eine beliebige Position um die Achse A gedreht. Damit der Patient vor dem Okular positioniert werden kann, wird die Kopfstütze 7 um denselben Winkel um den Gerätefuss 5 gedreht. Das Bedienungspanel 4 bleibt bei allen Bewegungen fest mit dem Gerätefuss 5 verbunden.

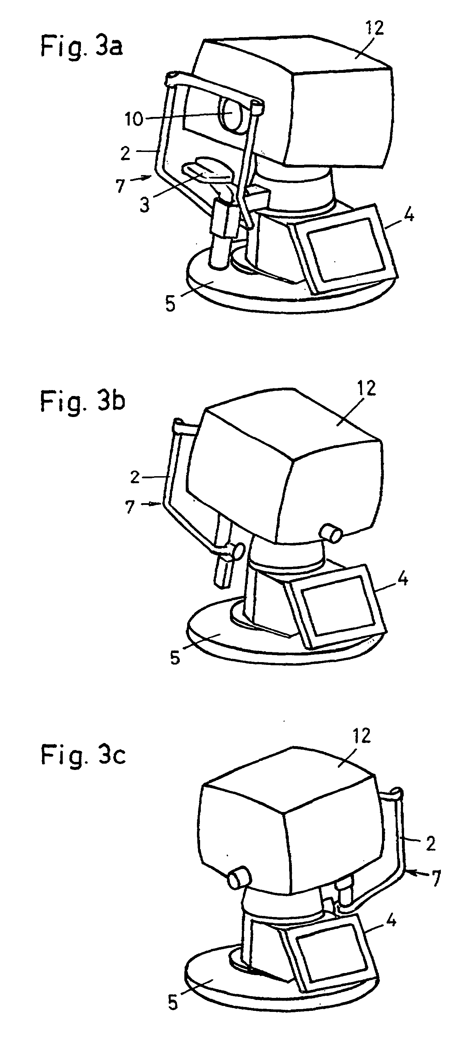

Bei der Anordnung gemäss Fig. 3a sind das Bedienungspanel 4, die Optikeinheit 12 mit dem Okular 10, sowie die Kopfstütze 7 so angeordnet, dass der Patient links neben der Bedienungsperson sitzt. Bei der Anordnung gemäss Fig. 3b sitzt der Patient wie bisher üblich, der Bedienungsperson gegenüber. Auch hier ist ein Feinpositionieren durch Bewegung der Optikeinheit 12 in allen drei Richtungen jederzeit möglich. Bei der Anordnung gemäss Fig. 3c sitzt der Patient rechts neben der Bedienungsperson. Ausser den in den Fig. 3a bis 3c genannten Positionen sind auch sämtliche Zwischenstellungen möglich.

## Patentansprüche

1. Perimeter für die Gesichtsfelduntersuchung, mit einer Kopfstütze (7), mit einem Bedienungspanel (4)zur Bedienung des Perimeters und zur Überwachung der Untersuchung und mit einem Gerätefuss (5) auf dem eine Optikeinheit (12) angeordnet ist, in der eine Untersuchungsoptik mit einem Okular (10) untergebracht ist, vor dem der Kopf des Patienten zu positionieren ist, **dadurch gekennzeichnet, dass** die Optikeinheit (12) auf dem Gerätefuss (5) beweglich gelagert und mittels wenigstens eines automatischen Antriebes (18, 19, 21) zur Feinpositionierung verstellbar ist, dass die Optikeinheit (12) um eine höhenverstellbare Säule (9) und in Richtung der Beobachtungsachse bewegbar ist, dass die Stirnstütze (6) Sensoren (8) zur Erkennung der Kopfposition aufweist, und dass die Kopfstütze (7) um den Gerätefuss drehbar ist, derart, dass die Position zwischen Patient und Bedienungsperson durch eine im wesentlichen beliebige Positionierung der Optikeinheit (12) und der Kopfstütze (7) frei wählbar ist.

2. Perimeter nach Anspruch 1, **dadurch gekennzeichnet, dass** die Optikeinheit (12) zur Feinpositionierung bezüglich des untersuchten Auges nachführbar ist.

3. Perimeter nach Anspruch 1 oder 2, **dadurch gekennzeichnet, dass** die Optikeinheit (12) mittels einer Hubbewegung vertikal verstellbar ist.

4. Perimeter nach einem der Ansprüche 1 bis 3, **dadurch gekennzeichnet, dass** die Optikeinheit (12) auf einer höhenverstellbaren Säule (9) beweglich gelagert ist.

## Claims

1. A perimeter for examining the field of vision comprising a head restraint (7), a control panel (4) for operating the perimeter and supervising the examination and with a unit base (5) on which an optical system (12) is located, which optical system (12) includes an optical examination system with an eyepiece (10) in front of which a patient's head is positioned, **characterized in that** the optical system (12) is movably mounted on the unit base (5) and is for precise positioning adjustable by at least one automatic drive (18, 19, 21), wherein the optical system (12) is rotatable around a column (9), that is vertically adjustable and can be moved in the direction of the vieweing axis, wherein the head rest (6) has sensors (8) to determine the position of the head, wherein the head restraint (7) is rotationally mounted on the unit base, wherein a position between a patient and an operator can be selected in essentially any desired positioning of the optical system (12) and of the head restraint (7).

2. The perimeter as claimed in claim 1, wherein a position of the optical system (12) can be tracked for precise positioning with respect to an eye being examined.

3. The perimeter as claimed in claim 1, wherein the optical system (12) is vertically adjustable by a vertical movement device.

4. The perimeter according to any one of the claims 1 to 3, wherein the optical system (12) is movably mounted on a vertically adjustable column (9).

## Revendications

1. Périmètre pour l'examen du champ visuel, comprenant un support pour la tête (7), avec un panneau de commande (4) pour commander le périmètre et pour contrôler l'examen et avec un une base d'appareil (5) sur laquelle est disposée une unité optique (12) dans laquelle est montée une optique d'examen avec un oculaire (10), devant laquelle doit venir se placer la tête du patient, **caractérisé en ce que** l'unité optique (12) est montée mobile sur la base d'appareil (5) et peut être réglée au moyen d'au moins un entraînement automatique (18, 19, 21) en vue d'un positionnement précis, **en ce que** l'unité optique (12) peut se déplacer autour d'une colonne réglable en hauteur (9) et dans la direction de l'axe d'observation, **en ce que** le support frontal (6) présente des capteurs (8) pour détecter la position de la tête, et **en ce que** le support pour la tête (7) peut tourner autour de la base de l'appareil de telle sorte que la position entre le patient et l'opérateur puisse être choisie librement par un positionnement essentiellement quelconque de l'unité optique (12) et du support pour la tête (7).

2. Périmètre selon la revendication 1, **caractérisé en ce que** l'unité optique (12) peut être orientée en vue d'un positionnement exact par rapport à l'oeil examiné.

3. Périmètre selon la revendication 1 ou 2, **caractérisé en ce que** l'unité optique (12) peut être réglée verticalement au moyen d'un mouvement de levage.

4. Périmètre selon l'une quelconque des revendications 1 à 3, **caractérisé en ce que** l'unité optique (12) est montée mobile sur une colonne (9) réglable en hauteur.
